# EUROPEAN PATENT SPECIFICATION

(11) **EP 1 342 448 B1**
(45) Date of publication and mention of the grant of the patent: **01.05.2013**
(21) Application number: 03003247.8
(22) Date of filing: 24.02.2003
(51) Int. Cl.: A61B 5/145

(54) **Minimum invasive optical format with integrated lance**
Minimal-invasives optisches Format mit integrierter Lanze
Format optique pour la chirurgie mini-invasive avec une lance intégrée

(30) Priority: 05.03.2002 US 361401 P
(43) Date of publication of application: 10.09.2003
(60) Divisional application: 10183150.1
(73) Proprietor: Bayer HealthCare LLC, Tarrytown, New York 10591 (US)
(72) Inventor: Dosmann, Andrew, J., Granger, Indiana 46530 (US)
(74) Representative: Linhart, Angela

(56) References cited:
- EP-A- 0 447 726
- DE-A- 3 708 031
- US-A- 2 830 587
- US-A- 6 041 246

## Description

### FIELD OF THE INVENTION

The present invention relates to minimum invasive techniques to determine compositions of body fluids. More particularly, the present invention relates to an optical format with a square fused silica lance for piercing skin of a user to harvest blood for testing.

### BACKGROUND OF THE INVENTION

Current methods of monitoring components of body fluids such as blood glucose involve painful skin punctures using steel needles having diameters from 28 (360 µm) to 24 (550 µm) gauge. Approximately thirty percent of steel lance skin punctures do not produce a blood sample thus requiring repeated puncturing. This increases the pain experienced by a patient causing some patients to avoid or skip testing. In order for prior art to conduct a proper analysis of a body fluid sample, a patient must lance and then manually harvest a minimum of 300 nL of body fluid such as blood into a format or a strip. An electrochemical or optical analysis of a chemical reaction is then performed to determine the levels of the desired component.

EP 044 7 726 A1 discloses body portion and a translucent needle portion projecting therefrom. US 5801057 A1 and US 2001/0001034 A1 each disclose a device for optical analysis of body fluids.

### SUMMARY OF THE INVENTION

The present invention is directed to a lance and optical format that can puncture the skin of a patient with little resulting pain, significantly improve reliability of blood production, automatically harvest a small blood sample, and analyze the sample with conventional transmission spectrometry. The lance is defined by a square, fused silica capillary tube. One end of the tube is cleaved to a sharp point that serves to pierce the skin of a patient. The other end of the lance is secured in a housing that has windows or aligned openings defining an optical path.

A blood sample is produced by first piercing a patient's skin followed by either vacuum or mechanical pressure around the wound to enhance blood flow. The lance is returned to a drop of blood at the puncture and the sample is harvested. Capillary action draws the sample into the lance. The sample reacts with reagents coated onto the walls inside the capillary, which produces a color change to the sample. Optical analysis of the sample can be performed using transmission spectrometry by passing a beam of light through the lance to a detector.

### BRIEF DESCRIPTION OF THE FIGURES

Other objects and advantages of the invention will become apparent upon reading the following detailed description and upon reference to the drawings in which:

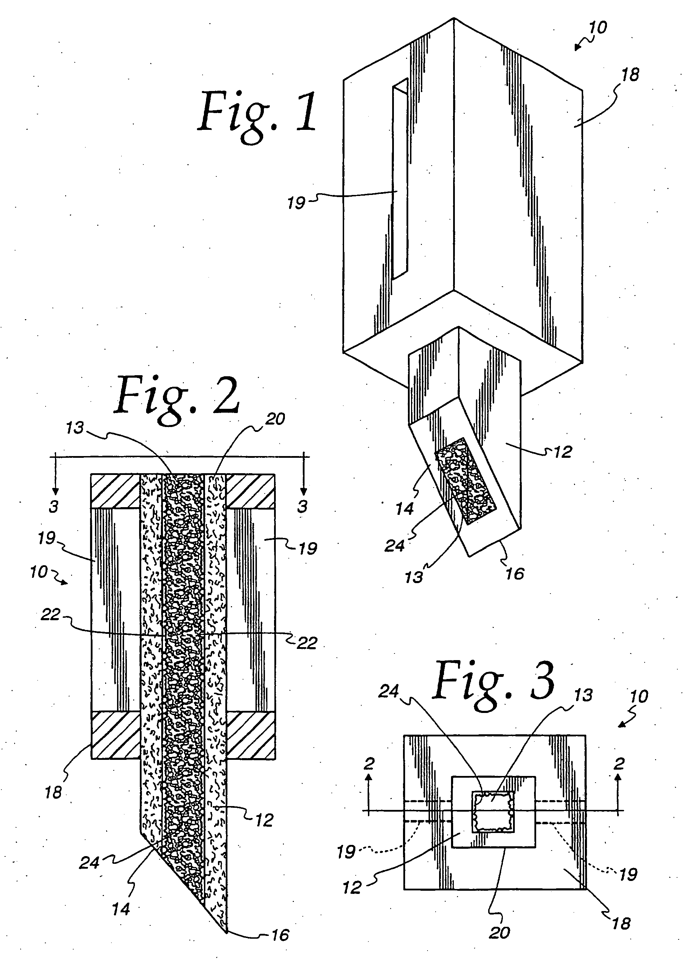

FIG. 1 is a perspective view of a minimum invasive optical format with an integrated lance constructed in accordance with the principles of the present invention;
FIG. 2 is a cross sectional view of the minimum invasive optical format with an integrated lance taken along line 2-2 of FIG. 3; and
FIG. 3 is a top plan view of the minimum invasive optical format with an integrated lance taken along line 3-3 of FIG. 2.

### DETAILED DESCRIPTION OF THE ILLUSTRATED EMBODIMENTS

Referring to the drawings, a minimum invasive optical format with an integrated lance generally designated by the reference numeral 10 is illustrated. The format with integrated lance 10 includes a lance 12 formed from a square fused silica capillary tubing manufactured by Polymicro Technologies of Phoenix, Arizona. The lance 12 can have other shapes such as rectangular depending on the intended use of the format with integrated lance 10.

The lance 12 is hollow and has a square channel 13 which can have a width of 50 µm, 75 µm or 100 µm and a length of 5 mm. These dimensions correspond to volumes of 13 nl, 29 nl, and 50 nl, respectively. A chemistry or reagent indicator 22 is dried onto an inside wall 24 of the channel 13. The chemistry 22 has an indicator formulation that is sensitive to an analyte being read. For example, if the analyte is glucose, the chemistry could be reductive hexokinase or glucose dehydrogenase. A first end 14 of the lance 12 is cleaved to a sharp point 16 at an angle of 45° ± 15° that serves to pierce the skin of a patient. The sharp point 16 at an angle of 45° ± 15° aids in cleanly cutting the skin as well as blood capillaries below the skin. Cutting blood capillaries improves the reliability of producing a blood sample to 98%.

To minimize pain when skin is pierced by the sharp point 16 of the lance 12, it is preferred that the outside dimension of the lance 12 be small. By using a square fused silica tube for the lance 12, the outside dimension of the lance 12 is 300 µm which is smaller than a similar dimension of a typical 28 gauge steel lance which has a diameter of 360 µm. Lance 10 can be modified depending on the intended use.

The format with integrated lance 10 also includes a housing 18 mounted on a second end 20 of the lance 12. The housing 18 has opposed windows or openings 19. The housing 18 controls the depth of a puncture into a patient's skin by the lance 12. The depth of a puncture corresponds to the length of the lance 12 extending out of the housing 18. In one embodiment, the portion of the lance 12 extending outside of the housing 18 measures 2 mm. The housing 18 also provides support for the lance 12 and resists breakage of the lance 12. Breakage of the lance 12, however, is minimized due to the strength of fused silica which is based on a Si-O bond which has a theoretical tensile strength of 2000 kpsi.

The optical format with integrated lance 10 is used to pierce the skin of a patient with the sharp point 16. Once a drop of blood appears at the puncture site, the tip or first end 14 of the lance 12 is returned to the drop of blood and capillary action draws a sample into the square channel 13. Another embodiment would leave the lance below the surface of the skin until capillary action or vacuum assisted capillary action obtains a sample. The sample is allowed to react with the dried chemistry 22 coated onto the capillary walls which produces a color change to the sample. The change in color is proportional to analyte concentration. The sample is then read with transmission spectrometry by passing a monochromatic collimated beam of light through the portion of the lance 12 in the housing 18 by passing the beam through one of the openings 19 in the housing 18. The opening 19 can be used to mask the beam down to only the sample area in the lance 12. A detector is located adjacent the other opening 19. The flat surfaces of the lance 12 provide an excellent optical window and the optical transmission of fused silica is spectrally flat from UV into infrared. Thus, readings can be done at several wavelengths to correct for interferences. For example, hematocrit levels in whole blood can interfere with glucose concentration determination. Hamatocrit could be determined at a wavelength that is independent of glucose. Glucose concentration can then be corrected. In addition, the square shape of the capillary 12 provides a two times increase in transverse optical interaction path length compared to round capillaries. The square shape of the lance 12 also provides alignment between the openings 19 in the housing 18 and the sample in the lance 12.

## Claims

1. A device (10) for the optical analysis of body fluids, comprising a lance (12) defined by a multisided tube wherein each side is planar and of a material that allows transmission of light,
a housing (18) mounted around a portion of said lance (12),
said housing (18) including openings (19) for passage of light through said housing (18) and said portion of said tube.

2. The device claimed in claim 1 wherein said tube is square.

3. The device claimed in claim 1 wherein said tube is rectangular.

4. The device claimed in one of the claims 1 to 3 wherein said tube includes a first end (14), said first end (14) being cleaved to a sharp point (16) for piercing skin.

5. The device claimed in one of the claims 1 to 4 comprising a reagent (22) on an inside surface (24) of said tube.

6. The device claimed in one of the claims 1 to 5 wherein said material is fused silica.

7. A method for making a device (10) for the optical analysis of body fluids comprising:
forming a square capillary tube of fused silica;
cleaving a first end (14) of said tube to form a sharp point (16); and
securing a housing (18) around a portion of said tube adjacent to a second end (20) of said tube, said housing (18) including openings (19) for passage of light through said housing (18) and said portion of said tube.

## Patentansprüche

1. Vorrichtung (10) für die optische Analyse von Körperflüssigkeiten mit
einer Lanzette (12), die durch eine Röhre mit mehreren Seiten definiert ist, wobei jede Seite planar ist und aus einem Material besteht, das den Durchgang von Licht gestattet,
einem Gehäuse (18), das um einen Abschnitt der Lanzette (12) montiert ist,
wobei das Gehäuse (18) Öffnungen (19) zum Durchgang von Licht durch das Gehäuse (18) und den Abschnitt der Röhre aufweist.

2. Vorrichtung nach Anspruch 1, wobei die Röhre quadratisch ist.

3. Vorrichtung nach Anspruch 1, wobei die Röhre rechteckig ist.

4. Vorrichtung nach einem der Ansprüche 1 bis 3, wobei die Röhre ein erstes Ende (14) aufweist, das zu einer scharfen Spitze (16) zum Durchstechen von Haut zugespitzt ist.

5. Vorrichtung nach einem der Ansprüche 1 bis 4, mit einem Reagens (22) an einer Innenfläche (24) der Röhre.

6. Vorrichtung nach einem der Ansprüche 1 bis 5, wobei das Material Quarzglas ist.

7. Verfahren zur Herstellung einer Vorrichtung (10) für die optische Analyse von Körperflüssigkeiten mit folgenden Schritten:
Bilden einer quadratischen Kapillarröhre aus Quarzglas,
Zuspitzen eines ersten Endes (14) der Röhre zur Bildung einer scharfen Spitze (16) und
Befestigen eines Gehäuses (18) um einen Abschnitt der Röhre in der Nähe eines zweiten Endes (20) der Röhre, wobei das Gehäuse (18) Öffnungen (19) zum Durchgang von Licht durch das Gehäuse (18) und den Abschnitt der Röhre aufweist.

## Revendications

1. Dispositif (10) pour l'analyse optique de liquides biologiques, comprenant
une lance (12) définie par un tube à plusieurs côtés, chaque côté étant plan et dans un matériau qui permet la transmission de la lumière,
un boîtier (18) monté autour d'une partie de ladite lance (12),
ledit boîtier (18) comportant des ouvertures (19) pour le passage de la lumière à travers ledit boîtier (18) et ladite partie dudit tube.

2. Dispositif selon la revendication 1 dans lequel ledit tube est carré.

3. Dispositif selon la revendication 1 dans lequel ledit tube est rectangulaire.

4. Dispositif selon l'une des revendications 1 à 3 dans lequel ledit tube comprend une première extrémité (14), ladite première extrémité (14) étant clivée en un point tranchant (16) pour percer la peau.

5. Dispositif selon l'une des revendications 1 à 4 comprenant un réactif (22) sur une surface intérieure (24) dudit tube.

6. Dispositif selon l'une des revendications 1 à 5 dans lequel ledit matériau est la silice fondue.

7. Procédé de fabrication d'un dispositif (10) pour l'analyse optique de liquides biologiques, comprenant :
la formation d'un tube capillaire carré de silice fondue ;
le clivage d'une première extrémité (14) dudit tube pour former un point tranchant (16) ; et
la fixation d'un boîtier (18) autour d'une partie dudit tube au voisinage d'une deuxième extrémité (20) dudit tube, ledit boîtier (18) comportant des ouvertures (19) pour le passage de la lumière à travers ledit boîtier (18) et ladite partie dudit tube.
